# EUROPEAN PATENT SPECIFICATION

(11) **EP 3 897 188 B1**
(45) Date of publication and mention of the grant of the patent: **29.11.2023**
(21) Application number: 19901280.8
(22) Date of filing: 19.12.2019
(51) Int. Cl.: C12N 1/12, C12N 1/22, A23K 50/75, A23K 10/30, A61P 33/02, A61K 36/02

(54) **ALGAL FEED INGREDIENT FOR CONTROLLING THE EFFECTS OF COCCIDIOSIS AND NECROTIC ENTERITIS IN POULTRY**
ALGENFUTTERMITTELINHALTSSTOFF ZUR BEKÄMPFUNG DER AUSWIRKUNGEN VON KOKZIDIOSE UND NEKROTISCHER ENTERITIS IN GEFLÜGEL
INGRÉDIENT ALIMENTAIRE ALGAL POUR CONTRÔLER LES EFFETS DES COCCIDIOSES ET DES ENTÉRITES NÉCROTIQUES CHEZ LES VOLAILLES

(30) Priority: 21.12.2018 US 201862783667 P
(43) Date of publication of application: 27.10.2021
(73) Proprietor: Zivo Bioscience, Inc., Keego Harbor, MI 48320 (US)
(72) Inventor: DAHL, Andrew A., Bloomfield Hills, MI 48302 (US); STEFFEK, Amy E., Royal Oak, MI 48067 (US); PFUND, William P., Kalamazoo, MI 49009 (US)
(74) Representative: Griffin, Philippa Jane
(86) International application number: PCT/US2019/067600
(87) International publication number: WO 2020/132318

(56) References cited:
- WO-A1-2017/142906
- WO-A1-2017/142906
- US-A1- 2013 305 599
- US-A1- 2017 106 027
- R LEVINE ET AL: "Evaluation of the effects of feeding dried algae containing beta-1,3-glucan on broilers challenged with Eimeria", POULTRY SCIENCE, vol. 97, no. 10, 1 October 2018 (2018-10-01), pages 3494-3500, XP055722067, Oxford ISSN: 0032-5791, DOI: 10.3382/ps/pey227
- HELENA M AMARO ET AL: "Antimicrobial activities of microalgae: an invited review", SCIENCE AGAINST MICROBIAL PATHOGENS: COMMUNICATING CURRENT RESEARCH AND TECHNOLOGICAL ADVANCES, 1 January 2011 (2011-01-01), pages 1272-1280, XP055410064,
- HELENA M AMARO ET AL: "Antimicrobial activities of microalgae: an invited review", SCIENCE AGAINST MICROBIAL PATHOGENS: COMMUNICATING CURRENT RESEARCH AND TECHNOLOGICAL ADVANCES, 1 January 2011 (2011-01-01), pages 1272-1280, XP055410063,
- LEVINE et al.: "Evaluation of the effects of feeding dried algae containing beta-1,3-glucan on broilers challenged with Eimeria", Poultry Science, vol. 97, no. 10, October 2018 (2018-10), pages 3494-3500, XP055722067,

## Description

### FIELD OF THE INVENTION

The present invention is set out in the appended claims and generally relates to farm animal health and specifically to an algal derived feed ingredient used to improve feed conversion rates and growth rates in poultry when poultry health is challenged by various conditions.

### BACKGROUND OF THE INVENTION

Coccidiosis is a parasitic disease affecting the intestines of farm animals, notably poultry. The parasite, of the genus *Eimeria,* affects animal growth and feed efficiencies, ultimately leading to death of the infected animals. The *Eimeria* species believed to infect poultry include *E. tenella, E. necatrix, E. acervulina,* and *E. maxima.* Infection begins with ingestion of oocysts, which undergo excystation to release thousands of sporozoites into the lumen of the intestine. For chickens, this process is believed to occur both enzymatically and through mechanical abrasion of the oocyst wall in the chicken's gizzard. A coccidiosis infection is just one factor, amongst other factors such as heat stress, feed restriction, various bacterial infections, and feed contaminants, which can weaken the quality of the tight junctions between epithelial cells in the lining of the intestinal tract of the animal and cause what's known as "leaky gut." The costs to the poultry industry in the United States associated with just coccidiosis exceeds $1.5 billion annually.

Aside from good animal husbandry, the only practical control of coccidiosis is to use anticoccidial drugs, such as sulfonamides, ionophores and toltrazuril. However, vaccination is costly, and recently some antibiotic resistance has been observed, (*see*, *e.g.*, N. Roth, et al., "The Application of Antibiotics in Broiler Production and the Resulting Antibiotic Resistance in Escherichia coli: A Global Overview," Poultry Science, 0:1-14, (2018)).

Necrotic enteritis is another common disease in poultry. Necrotic enteritis is caused by a *Clostridium perfringens* infection, characterized by severe necrosis of the intestines. C. *perfringens* is a gram-positive bacterium, capable of producing up to 17 different toxins. Infected animals die very quickly, e.g., within 24 hours, making treatment of the disease, once manifested, impractical. Typically, necrotic enteritis is prophylactically prevented with antibiotics such as oxytetracycline dihydrate (OTC), doxycycline hydrochloride, amoxicillin, and the like. Although entirely unrelated to coccidiosis, the two diseases do appear together, and present a huge challenge to the poultry industry. Controlling the two diseases simultaneously has resulted in extensive use of antibiotics as prophylactics in the poultry industry.

In view of Europe's ban on the use of antibiotics as growth promoters in animal feed, alternatives to antibiotics in the prevention of necrotic enteritis are needed. To that end, some research has been reported on the use of probiotics, plants, essential oils, and enzymes for the prevention of necrotic enteritis in chickens, (*see*, *e.g.*, D. L. Caly, et al., "Alternatives to Antibiotics to Prevent Necrotic Enteritis in Broiler Chickens: A Microbiologist's Perspective," Front. Microbiol., 6, 1336, (2015)).

Despite this research into natural coccidiostats and alternatives to antibiotics to prevent necrotic enteritis, there remains the need to discover new and natural feed ingredients for use in the poultry industry. Ideally what is still needed is a natural feed ingredient capable of maintaining good feed conversion rates and growth rates for birds challenged by these common pathogens or other stress factors such as feed restriction, heat stress and feed contaminants.

WO 2017/142906 describes Nutritional Support for Animals via Administration of an Algal Derived Supplement. Levine, et al., (2018) Poult. Sci. 97(10): 3494-3500, describes Evaluation of the effects of feeding dried algae containing β-1,3-glucan on broilers challenged with Eimeria.

### SUMMARY OF THE INVENTION

The invention provides a poultry feed composition including an algal biomass comprising a species of *Klebsormidium* algae for use in a method of mitigating symptoms relating to coccidiosis or necrotic enteritis in poultry, as set out in the appended claims.

It has now been discovered that an algal biomass can be used in poultry feed as a natural feed ingredient to maintain or enhance feed conversion rates and growth rates in poultry that are challenged by coccidiosis and necrotic enteritis. A novel and nonobvious use of an algal biomass in maintaining poultry health in the presence of coccidiosis and necrotic enteritis is herein disclosed.

Algal biomass augmented poultry feed was shown to reverse the detrimental effects caused by pathogen challenges in chickens. Notably, good feed conversion rates were maintained in birds challenged by the common *Eimeria* and *Clostridium* pathogens. Lesion scores were significantly improved relative to what was seen in pathogen-challenged control birds. Further, the improvement in lesion scores seen by using the algal biomass as a nutritional ingredient in feed were comparable to lesion scores seen with the Coban^{®} antibiotic additive (monensin, USP) from Elanco. Hence, the algal biomass disclosed herein augments poultry feed for the maintenance and enhancement of poultry health.

An algal biomass can maintain or enhance feed conversion rates and growth rates in poultry challenged by various farming practices and environmental conditions such as, for example, feed restriction, heat stress, environmental contaminants and feed contaminants. For example, a poultry feed composition comprising an algal biomass obtained from at least one species of *Klebsormidium* algae may reduce symptoms of pathogenic infections, farming practices, or environmental stresses, wherein the symptoms include weight loss, elevated/abnormal feed conversion rate (FCR), elevated/abnormal mortality rates, and intestinal lesions.

In all embodiments of the invention, the algal biomass comprises algae from the genus *Klebsormidium.* The algal biomass may be obtained by processing the biological material of NCMA Deposit # PATENT201602001. For example, an algal biomass comprising a species of *Klebsormidium* algae may be obtained by culturing the microorganisms in NCMA Deposit #PATENT201602001 to a processable mass of plant material and separating liquid from the plant material to obtain the algal biomass.

An algal biomass for use as a nutritional ingredient in poultry feed comprising at least one species of *Klebsormidium* is disclosed herein but does not form part of the present invention. The algal biomass may comprise a species of *Klebsormidium* selected from the group consisting of *K. acidophilum, K. bilatum, K. crenulatum, K. dissectum, K. drouetii, K. elegans, K. flaccidum, K. fluitans, K. fragile, K. klebsii, K. lamellosum, K. montanum, K. mucosum, K. nitens, K. pseudostichococcus, K. scopulinum, K. sterile, K. subtile, K. subtilissimum, K.* tribonematoideum, and mixtures thereof.

An algal biomass for use as a nutritional ingredient in poultry feed comprising a species of *Klebsormidium* selected from the group consisting of *Klebsormidium nitens, Klebsormidium flaccidum,* and mixtures thereof is disclosed herein but does not form part of the present invention. For example, an algal biomass for use as a nutritional ingredient in poultry feed may comprise *Klebsormidium nitens* or *Klebsormidium flaccidum.* In certain examples, an algal biomass for use as a nutritional ingredient in poultry feed may comprise *Klebsormidium flaccidum.*

An algal biomass for use as a nutritional ingredient in poultry feed may be obtained by culturing freshwater algae to obtain plant material, separating the plant material from the liquid, optionally mechanically pressing out excess water from the cellular material, and then drying the plant material to obtain a dried algal biomass. The drying process may consist of any combination of ambient drying, air drying, spray drying, freeze drying or heated drying.

A dried algal biomass may be obtained by skimming or surface pumping algae material from a raceway pond consisting of continuously cultivated *Klebsormidium* algae, draining off liquid from the algae to obtain a moist algal biomass, and then spray drying the moist algal biomass to obtain the dried algal biomass.

In various embodiments, a dried algal biomass may be incorporated into poultry feed as a nutritional ingredient at levels of from about 0.01 wt.% (0.2 pounds dried algal biomass/ton feed) to about 1.0 wt.% (20 pounds dried algal biomass/ton feed), based on the total weight of the poultry feed composition.

For example, a dried algal biomass may be incorporated into poultry feed as a nutritional ingredient at levels of from about 0.025 wt.% (0.5 pounds dried algal biomass/ton feed) to about 0.55 wt.% (11 pounds dried algal biomass/ton feed), based on the total weight of the poultry feed composition.

For example, a dried algal biomass may be incorporated into poultry feed as a nutritional ingredient at levels of from about 0.10 wt.% (2.0 pounds dried algal biomass/ton feed) to about 0.20 wt.% (4.0 pounds dried algal biomass/ton feed), based on the total weight of the poultry feed composition.

For example, a dried algal biomass may be incorporated into poultry feed as a nutritional ingredient at levels of from about 0.125 wt.% (2.5 pounds dried algal biomass/ton feed) to about 0.175 wt.% (3.5 pounds dried algal biomass/ton feed), based on the total weight of the poultry feed composition.

The remainder of the poultry feed that is not algal biomass may comprise a mixture of ingredients suitable for use in poultry or another animal feed. For example, the non-algal portion of the poultry feed herein may comprise cereals, proteins, fats, and oils. In various examples, a poultry feed may comprise a corn-soy base or a wheat-soy base, such as determined by country of origin.

The algal biomass augmented poultry feed disclosed herein may be in the physical form of a dry mash feed or a pelleted feed, wherein the pelleted feed may be ground to provide crumbles of material for feeding young birds, or another physical form of animal feed suitable for use in the poultry industry. The dry mash feed can be converted to the pelleted feed by extrusion of the mash feed through a suitable die

The invention provides a poultry feed composition for use in a method of mitigating the symptoms of coccidiosis or necrotic enteritis in poultry, the poultry feed composition including an algal biomass comprising a species of *Klebsormidium* algae. In various embodiments, a poultry feed comprises *Klebsormidium nitens* or *Klebsormidium flaccidum.* The symptoms exhibited in the poultry in need thereof include at least one of elevated feed conversion rate, reduced growth rate, increased mortality rates and increased intestinal lesions.

In various embodiments, the poultry feed for mitigating symptoms of coccidiosis or necrotic enteritis in poultry comprises (a) an algal biomass derived from at least one species of *Klebsormidium* algae; and (b) at least one of a cereal, protein, fat and oil. In various embodiments, the poultry feed comprises *Klebsormidium nitens* or *Klebsormidium flaccidum.* The symptoms exhibited in the poultry in need thereof include at least one of elevated feed conversion rate, reduced growth rate, increased mortality rates and increased intestinal lesions.

A method of producing an algae-enhanced poultry feed is disclosed herein but does not form part of the present invention. The method comprises: (a) culturing at least one species of *Klebsormidium* algae to obtain an algal biomass; (b) optionally removing water from the algal biomass and optionally drying the algal biomass; and (c) blending the algal biomass into a poultry feed to provide the algae-enhanced poultry feed. For example, the culturing of at least one species of Klebsormidium algae comprises a continuously cultivated raceway pond consisting of Klebsormidium *nitens* or Klebsormidium *flaccidum.* For example, the step of drying may comprise spray drying.

### DETAILED DESCRIPTION OF THE INVENTION

An algal biomass, derived from a single algal species or from multiple algae species, when incorporated into poultry feed as a nutritional feed ingredient and fed to poultry animals, promotes general health in the poultry by improving feed conversion rates and growth rates in animals challenged by coccidiosis and/or necrotic enteritis. In various examples, an algal biomass augmented poultry feed reverses detrimental effects in the animals caused by the *Eimeria* and *Clostridium* pathogens. A poultry feed augmented with an algal-derived nutritional feed ingredient may mitigate the symptoms caused by stresses in poultry animals, such as feed restriction, heat stress, bacterial or other forms of infection, feed contaminants, or environmental contaminants.

All embodiments of the invention involve at least one species of *Klebsormidium* algae. The algal biomass may be obtained by culturing biological material from NCMA Deposit # PATENT201602001 and separating out the plant material. The algal biomass may be obtained by harvesting algal material from open ponds, e.g., raceways, designed to grow a *Klebsormidium* species of algae.

In general, an algal biomass, when incorporated as a nutritional ingredient in poultry feed, has been shown to maintain good feed conversion rates (FCR) for birds challenged by the *Eimeria* and *Clostridium* pathogens.

In various studies, an algal biomass as disclosed herein reduced overall FCR at all levels of inclusion of the algal biomass in the feed, compared to a pathogen-challenged group. Birds fed the algal biomass ingredient had similar FCR to the unchallenged control group of birds. Percent necrotic enteritis mortality was significantly reduced in birds fed the algal biomass. Mortality in a non-medicated challenged group was 25% versus 5% in a group of pathogen-challenged birds fed a poultry feed having the algal biomass ingredient at 0.551 wt.% incorporation.

In other studies, overall FCR was reduced in the algal biomass fed group compared to the challenged control group. Birds fed the algal biomass augmented feed had similar FCR to a salinomycin (i.e., medicated) treatment group. Birds fed the algal biomass augmented feed had significant reduction in lesion score versus the pathogen-challenged control group of animals.

### Definitions and Abbreviations

The term "poultry" is used herein to refer to all domesticated fowl used for human or non-human animal consumption, including, for example, chickens, hens, roosters, turkeys, ducks, quail, and geese.

The term "poultry feed" is used herein to refer to commercial animal feed used in poultry farming, which is typically corn/soy-based in the U.S. and wheat-based in other parts of the world such as Europe. A poultry feed herein, to be augmented with an algal biomass, may be a "starter feed" for use during about the first two weeks of growth, a "grower feed" for use during about the next two weeks of growth, or a "finisher feed" for use for about two additional weeks of growth thereafter. These three feeds tend to vary in protein levels so as to be appropriate for poultry in one of these three stages of growth.

As it relates to poultry feed, the term "mash" refers to a powdered feed, which is essentially dry and which has the appearance of a fine corn meal. This physical form of dry feed, which is not wet and should not be confused with "mush," is used to feed 1-2 day old chicks that are too young to handle pelleted feed. Further, mash feed is readily convertible into pelleted feed simply by extrusion through a small orifice die and allowing the extruded "worms" to break into pellets. Pelleted feed may be crumbled for feeding to young birds incapable of eating pellets.

The term "algal biomass" is used herein to generally refer to cellular material (e.g. whole algal cells) derived from naturally occurring or genetically modified algae ("GMO algae"). For example, an algal biomass may comprise dried or otherwise desiccated algae of one or more species of algae. A dried algal biomass may optionally comprise powdered or granulated desiccated algae from one or more species of algae. An exemplary algal biomass may be derived from an algal culture comprising a *Klebsormidium* species of algae by separating and drying the cellular material and optionally granulating, powdering, or milling the cellular material obtained upon drying. An algal biomass may be obtained by processing the biological material of NCMA Deposit # PATENT201602001, such as, for example, by culturing the microorganisms in tanks or open raceways to produce a processible amount of plant material, draining and/or compressing the plant material to remove liquid components, and then drying the remaining cellular material by any convenient method, such as spray drying or freeze drying, to obtain the dried algal biomass that is usable as a nutritional poultry feed ingredient.

The term "NM, No CP" is used herein to denote a non-medicated group of birds (i.e., no antibiotics) that were not challenged with *C. perfringens* (denoted *C. perfringens* (-)), and were healthy.

The term "NM, CP" is used herein to denote a non-medicated group of birds (i.e., no antibiotics) that were challenged with *C. perfringens* (denoted *C*. *perfringens* (+)), and were diseased. In no case does "CP" indicate that *C. perfringens* was part of the poultry feed. The term CP indicates birds were challenged with *C. perfringens* to induce disease.

The term "Product Z 0.039%, CP" is used herein to denote a poultry feed augmented with the algal biomass ingredient at the level of 0.039 wt.%, based on the total weight of the feed, and a group of birds challenged with *C. perfringens* (i.e., *C*. *perfringens* (+)) to induce disease.

The term "Product Z 0.077%, CP" is used herein to denote a poultry feed augmented with the algal biomass ingredient at the level of 0.077 wt.%, based on the total weight of the feed, and a group of birds challenged with *C. perfringens* (i.e., *C*. *perfringens* (+)) to induce disease.

The term "Product Z 0.551%, CP" is used herein to denote a poultry feed augmented with the algal biomass ingredient at the level of 0.551 wt.%, based on the total weight of the feed, and a group of birds challenged with *C. perfringens* (i.e., *C*. *perfringens* (+)) to induce disease.

The acronym "DOT" is used herein to denote "day of treatment," which is the number of days from the start of a study when a desired feeding composition is administered to a poultry animal. DOTs herein may be 0, 12, 14, 21, or 28 days, for example.

The acronym "FCR" is used herein to denote "feed conversion rate" or "feed conversion ratio," which is the rate at which feed intake is converted to weight gain in the animal. For example, today's broilers weigh about 5-1/2 pounds at 39 days, with a live-weight normal feed conversion ratio of about 1.6. Diseased animals show a higher feed conversion ratio, meaning the animal needs to consume more feed to gain weight, (i.e., the animal is less efficient).

The acronym "GIT" is used herein to denote gastrointestinal track of a poultry animal.

The term "No-Additive, Non-Infected" is used herein to denote normal diet, and non-infected animals.

The term "No-Additive, Infected" is used herein to denote normal diet, and infected animals.

The term "salinomycin, 60 g/t" is used herein to denote normal diet, infected animals, treated with antibiotic.

The term "Product Z, 0.551%" is used herein to denote a poultry feed product containing 0.551 wt.% algal biomass, based on the total weight of the feed.

### The algal biomass

An algal biomass may comprise whole algal cells of one or more algal cultures grown in distilled water or freshwater supplemented with various nutrients. A deposit of biological material that can be used to obtain an algal biomass for use as a nutritional ingredient in poultry feed was originally deposited at the Provasoli-Guillard National Center for Marine Algae and Microbiota - Bigelow Laboratory for Ocean Sciences, (NCMA, 60 Bigelow Drive, East Boothbay, Maine 04544 U.S.A.) on October 6, 2006, converted to a deposit made under the provisions of the Budapest Treaty on February 12, 2016, and assigned by the International Depositary Authority the accession # PATENT201602001. This deposit is available to the public upon grant of a patent disclosing the same. This deposit was made pursuant to 37 C.F.R. §1.808 and MPEP §2410.01 and, therefore, access to the deposit will be available during pendency of this application making reference to the deposit to one determined by the Commissioner to be entitled thereto under 37 C.F.R. §1.14 and 35 U.S.C. §122 and with one exception, that all restrictions imposed by the depositor on the availability of the deposited biological material to the public be irrevocably removed upon the granting of the patent.

A method of producing an algal biomass for use as a nutritional ingredient in poultry feed for maintaining or improving feed conversion rates and growth rates in animals challenged by coccidiosis and/or necrotic enteritis comprises obtaining a sample of the biological material of NCMA Deposit # PATENT201602001, culturing the material to obtain processible quantities of plant material, and separating the solid from the liquid components to obtain the algal biomass and a supernatant respectively. The algal biomass may then be dried prior to its use as a poultry feed ingredient, such as by spray drying.

The algal biomass may then be formulated into a poultry feed composition comprising the algal biomass as an ingredient, such as, for example, by blending the algal biomass with typical non-algal poultry feed ingredients such as cereals, proteins, fats and oils. The resulting blended material may then be fed to animals as is, e.g. in the form of a mash. In other embodiments, the resulting blended material may be pelleting using pelleting machinery like an extruder. These aspects are discussed in more detail below.

In certain examples, an algal biomass is obtained from a *Klebsormidium* species of algae by compressing the liquid from the *Klebsormidium* species of algae and further desiccating the remaining solid plant material to produce the algal biomass. Furthermore, a sample of a culture obtained from the biological material of NCMA Deposit #PATENT201602001 may be physically compressed to squeeze out supernatant from the cellular material and then dried to produce an algal biomass in accordance to the present disclosure.

An algal biomass for use as an ingredient in a poultry feed in accordance with the invention includes a species of algae from the genus *Klebsormidium*. *Klebsormidium* is a genus of filamentous charophyte green algae of 20 known species, including *K. acidophilum*, *K. bilatum*, *K. crenulatum*, *K. dissectum*, *K. drouetii, K. elegans, K. flaccidum*, *K. fluitans, K. fragile*, *K. klebsii*, *K. lamellosum*, *K. montanum*, *K. mucosum*, *K. nitens*, *K. pseudostichococcus*, *K. scopulinum*, *K. sterile*, *K. subtile*, *K. subtilissimum*, and *K*. *tribonematoideum.* In various embodiments, an algal biomass comprises any presently known, or yet to be discovered, species of *Klebsormidium* algae.

For example, an algal biomass may comprise an algae species selected from the group consisting of *K. acidophilum*, *K. bilatum*, *K. crenulatum*, *K. dissectum*, *K. drouetii*, *K. elegans*, *K. flaccidum*, *K. fluitans*, *K. fragile*, *K. klebsii*, *K. lamellosum*, *K. montanum*, *K. mucosum, K. nitens, K. pseudostichococcus, K. scopulinum, K. sterile, K. subtile, K. subtilissimum*, *K.* tribonematoideum, and mixtures thereof.

Furthermore, an algal biomass may comprise an algae species selected from the group consisting of *Klebsormidium nitens*, *Klebsormidium flaccidum*, and mixtures thereof. In various embodiments, an algal biomass may comprise *Klebsormidium nitens.* Also, an algal biomass may comprise *Klebsormidium flaccidum.*

### Poultry feed augmented with the algal biomass ingredient

The algal biomass described above may be incorporated into a poultry feed composition at levels of from about 0.01 wt.% to about 1.0 wt.%, based on the total weight of the poultry feed composition (0.2 to 20 pounds dried algal biomass/ton feed). For example, a dried algal biomass may be incorporated at levels of from about 0.025 wt.% to about 0.55 wt.%, based on the total weight of the poultry feed composition (0.5 to 11 pounds dried algal biomass/ton feed). For example, a dried algal biomass may be incorporated at levels of from about 0.10 wt.% to about 0.20 wt.%, based on the total weight of the poultry feed composition (2.0 to 4.0 pounds dried algal biomass/ton feed). For example, a dried algal biomass may be incorporated at levels of from about 0.125 wt.% to about 0.175 wt.%, based on the total weight of the poultry feed composition (2.5 to 3.5 pounds dried algal biomass/ton feed). In certain examples, the incorporated amount of dried algal biomass in poultry feed is about 11 pounds/ton of feed. In certain examples, the incorporated amount of dried algal biomass in poultry feed is about 3.5 pounds/ton of feed. Exemplary incorporation levels are detailed in the Definitions and Abbreviations section above, and in the Examples below.

The other ingredients in the poultry feed, i.e., the non-algal ingredients, may comprise ingredients typically used for animal feed in the poultry industry of the country of interest, such as the U.S. The non-algal ingredients may be selected from the group of feed ingredients consisting of cereals, fats, oils, proteins, and mixtures thereof. Cereals may comprise any combination of barley, corn, sorghum, or wheat, and the like, or cereal byproducts such as grain hulls, bran, germ, gluten, gluten meal, middling, grain screenings, mixed screenings, groats, mill run, corn hominy, barley malt sprouts, rice polishing, wheat red dog, and wheat shorts. Fats and oils may comprise any combination of tallow, lard, white grease, poultry fat, animal fat, yellow grease, and vegetable fat. Protein sources may include canola, fish meal, field peas, meat and bone meal, soybeans and some of the cereal byproducts listed above. Further non-limiting examples of poultry feed that may be used as the base material for the algal biomass augmented feed disclosed herein may be found in various periodicals, such as, "Animal Feed Science and Technology," co-editors V. Mlambo, B. Mullan and A. K. Patra, Elsevier publisher, and "Journal of Applied Animal Nutrition," Cambridge University Press publisher.

Specific poultry feeds for augmenting with an algal biomass may optionally be formulated for feeding poultry, such as broilers, at particular growth stages, namely, starter, grower or finisher. Thus, a poultry feed supplemented with an algal biomass nutritional ingredient may be appropriate for a starter diet, a grower diet, or a finisher diet.

### EXAMPLES

In general, studies were designed to compare algal biomass augmented poultry feed to control poultry feed not having the algal biomass ingredient, and to feed supplemented with the Coban^{®} antibiotic additive. Studies included groups of animals that were pathogen challenged to induce disease along with control groups that were not pathogen challenged. Poultry feed, with or without the algal biomass ingredient or the antibiotic additive were in the form of a mash or pellets, or crumbles derived from pellets. The endpoints measured were weight gain, feed conversion rate (FCR), mortality, and intestinal lesion scores. These endpoints are part of the symptoms seen in poultry with coccidiosis or necrotic enteritis.

The results generally showed that, as expected, the pathogen challenge caused the deleterious symptoms of decreased weight gain, increased FCR, increased mortality, and increased number of intestinal lesions compared to the unchallenged control group. However, by including the algal biomass as an ingredient in the poultry feed, the detrimental symptoms caused by the pathogen challenge in the primary endpoints were reversed. In particular;
1. Weigh gain and FCR for birds fed the algal biomass augmented feed were comparable to the unchallenged control;
2. Mortality for birds fed the algal biomass augmented feed was comparable to the unchallenged control;
3. Lesion scores were significantly improved for birds fed the algal biomass augmented feed relative to what was seen in pathogen-challenged birds that did not receive any feed additive, but not as low as baseline scores; and
4. The improvement in lesion scores were comparable to what was seen with the Coban^{®} antibiotic additive.

The overall conclusion is that algal biomass, when used as a natural ingredient to poultry feed, performed at least as well as the commercial antibiotic in protecting a flock from the deleterious effects of the pathogens. The algal biomass augmented poultry feed provided control of the symptoms relating to coccidiosis and necrotic enteritis in poultry.

### Example 1. Preparation of an algal biomass (single culture to small culture)

A sample of NCMA Deposit # PATENT201602001 was used to grow algae axenically on an mBBM agar petri plate. A single colony was picked from the plate and grown in mBBM media in a shake flask for one month to achieve adequate density for roux bottle inoculation. Algae inoculum grown in roux bottles was progressively divided to produce the minimum inoculation density required for panel reactor growth. Algae biomass grown in reactors was placed in a 25 µm filter sock to remove supernatant using gravity flow. Excess supernatant was removed with manual squeezing of the filter sock until the consistency of the algal biomass reached a thick paste. The algal biomass was then spread into a ½ inch layer on a stainless steel tray and placed at -80° C until frozen. The tray was then moved to a vacuum equipped freeze-drier, and the material held at -50° C to -60° C for several days until the moisture level was reduced to about 5 to 10% by weight. The freeze-dried algal biomass was stored in a refrigerator at 4° C or in a freezer at -20° C until use.

### Example 2. Preparation of an algal biomass (scale-up of small culture)

Monocultures of a filamentous *Klebsormidium* alga are grown in 120 L and 240 L flat panels with a 4-inch light path, 3-9 mM NO₃ in mBBM media, and CO₂/air mixing or within outdoor, covered, HEPA filtered ponds using natural light. Harvesting consist of drawing off the liquid and algae that is contained in the liquid and separating the liquid from the algal biomass. There is typically about 1.4 grams of algae per liter of water. Upon harvesting, biomass is placed in a 25 µm filter sock to remove water using gravity flow. Excess water is removed with manual squeezing resulting in a wet paste that is about 15% solids and 85% water. The appearance of the biomass is that of a bright green thick paste. On average, about 8-10 kg wet weight yields about 1 kg of algal biomass in the form of a paste. This material is then spread onto stainless steel trays in a ½" layer and placed in a -80° C freezer until frozen or ready for further processing. Freeze dried biomass is achieved by placing the tray in a freeze-drier equipped with a vacuum pump and set at -50° C to -60° C for several days until the moisture level was reduced to 5-10%. Freeze-dried biomass was stored in a refrigerator at 4° C or freezer at -20° C until use. The freeze dried algal biomass material produced in accordance to this method effectively has no water and is in the form of a dark green powder or a brittle cake. This resulting material is then blended with other feed ingredients at the levels desired to form the algal biomass augmented poultry feed. The blended feed in the form of a mash feed may then be pelleted through an extruder to obtain pelleted feed.

### Example 3. Preparation of an algal biomass by continuous cultivation in raceway ponds

Alternatively, green, freshwater filamentous microalgae derived from *K. flaccidum* are constantly cultivated in open-air, partially open-air and/or greenhouse-covered raceways exposed to natural sunlight. The raceways ponds are typically from 20-30 cm in depth. The raceways are inoculated with the *Klebsormidium* algae, along with a micronutrients and macronutrients suitable for the species of alga. CO₂ may be added to the ponds as a gas bubbled in, or in a dry form such as calcium carbonate. When the density of the algae reaches about 0.8 to about 1.0 grams algae per liter, the algae are harvested every six to eight days. Harvesting consisted of skimming off the algae with skimmer or surface pumps, rinsing the algae with potable water and then centrifuging the algae to separate the biomass from the supernatant. drawing off an algae liquid suspension and separating the liquid from the plant material to obtain the algal biomass. The moist, isolated algae may be frozen -25° C until needed for production, or dried into the algal biomass for use in poultry feed by spray drying. If frozen, the previously frozen material may be thawed, rinsed with potable water, and then spray dried. Harvested algae can also be gravity feed over separation screens, and then rinsed to remove minerals, etc. Spray drying can be conducted at from about 370° to about 620° C to obtain dried algal biomass for use in poultry feed. For this purpose, a BUCHI mini spray dryer B-290 or other suitable spray dryer is used. The moisture level target for the spray dried algal biomass is less than about 10 wt.%, and in various embodiments, less than about 5 wt.% residual moisture. The bright green powder obtained in this way has at least six months stability under ambient conditions (25° C and 60% relative humidity). This resulting material is then blended with other feed ingredients at the levels desired to form the algal biomass augmented poultry feed. The blended feed in the form of mash feed may then be pelleted through an extruder to obtain pelleted feed.

### Example 4. Comparative efficacy of algal biomass augmented feed administered for the control of necrotic enteritis (NE) caused by Clostridium perfringens in broiler chickens Experimental design

The study consisted of 40 cages starting with 9 chicks in each cage. The feedings were replicated in 8 blocks of 5 cages each. In this example, "Product Z" refers to poultry feed containing the algal biomass ingredient at 0.039 wt.%, 0.077 wt.% or 0.551 wt.%, as indicated, based on the total weight of the poultry feed.

TABLE 1 below summarizes the feed groups.

**TABLE 1: Feed Groups**

| | | Coccidial Challenge | *Clostridium perfringens* | Cages per group |
|---|---|---|---|---|
| 1 | Nonmedicated | DOT 14 | No | 8 |
| 2 | Nonmedicated | DOT 14 | DOT 19, 20, and 21 | 8 |
| 3 | Product Z | DOT 14 | DOT 19, 20, and 21 | 8 |
| | feed inclusion rate 0.039 wt.% | | | |
| 4 | Product Z | DOT 14 | DOT 19, 20, and 21 | 8 |
| | feed inclusion rate 0.077 wt.% | | | |
| 5 | Product Z | DOT 14 | DOT 19, 20, and 21 | 8 |
| | feed inclusion rate 0.551 wt.% | | | |

### Materials and Methods:

### A. Feeds

An unmedicated commercial chicken feed composition compounded with feedstuffs commonly used in the U.S. poultry industry, such as cereals and proteins, was obtained. The feed was representative of U.S. commercial feeds and the calculated analyses met or exceeded NRC broiler feed requirements. Experimental feeds augmented with the algal biomass were prepared from this commercial poultry feed. The various feeds were mixed to assure a uniform distribution of algal biomass into the mash feed. The mixer was flushed between batches to prevent cross contamination between batches. The feed was transferred to the respective buildings and distributed among cages belonging to the same feed group. This ration in mash form was fed to the poultry throughout the study.

### B. Animals

Day of hatch male broiler chicks of strain Cobb 500 were obtained from Cobb-Vantress, Cleveland, GA, and only healthy appearing chicks were used in the study.

### C. Housing

Upon arrival, chicks were raised in Petersime battery cages. At placement the birds were fed the various feeds. The Petersime batteries were in an insulated building having a concrete floor and a metal overhead structure. The feeder/water space per bird was 9 birds per 24 x 3.5-inch feeder per water trough. Uniform temperature and illumination was provided.

### Procedures

### A. Bird Allocation and Cage Randomization

Cages were blocked by location in the battery. The study began when the birds were placed (day of hatch, i.e., DOT 0) at which time they were allocated to the experimental cages. No birds were replaced during the study.

### B. Cage Weights

All birds were weighed on DOT 0, 14, 21, and 28. Feed was weighed in on DOT 0 and remaining feed was weighed on DOT 14, 21, and 28.

### C. Disease Induction

On DOT 14, all birds were orally inoculated with ~5,000 oocysts of *E. maxima.* Starting on DOT 19, all birds, except feed group 1 were given a broth culture of *C*. *perfringens* ∼10⁸ cfu/ml. The birds were administered a fresh broth culture once daily for 3 days (on DOTs 19, 20, and 21).

### D. Necrotic Enteritis Intestinal Lesion Scoring

On DOT 21, three birds from each cage were selected, sacrificed, weighed, and examined for the degree of presence of Necrotic Enteritis lesions. The scoring was based on a 0 to 3 score, with 0 being normal and 3 being the most severe.

### E. Intestinal Tract Collection

On DOT 28, one bird from each cage was selected, sacrificed, weighed, and the entire portion of the GIT was collected. Samples were individually packaged and shipped same day overnight on ice to: Agro BioSciences, Attn: Evan Hutchison, 10437 Innovation Dr., Wauwatosa, WI 53226.

### F. Gut Cross Section

On DOT 28, one bird from each cage was selected, euthanized, and a small cross section of intestine was cut and frozen. The cross sections were stored in a freezer.

### Management

1. The facility was checked thoroughly to assure that all cages had water and that feed was available in every cage. The building temperature's range was maintained at an appropriate temperature for the age of the birds.
2. Uniform and continuous illumination was provided by fluorescent lamps hung vertically along the wall.
3. Feed and water were given *ad libitum,* noting that the inclusion rate of algal biomass was fixed.
4. Cages were checked twice daily. Observations included were the availability of feed and water, temperature control, and any unusual conditions. The birds were watched closely for any abnormal reactions.
5. When mortality birds were removed from cages, the cage number, date, weight of the bird, sex, and probable cause of death were recorded.

### Data Analysis

Means for cage weight gain, feed consumption, feed conversion rate, NE lesion scores, and % NE mortality were calculated.

### Results and Conclusions

TABLE 2 below shows the results of the study. The conclusion is that overall FCR was reduced for all algal biomass product inclusion level feeds compared to the challenged group. Birds fed the algal biomass product had similar FCR to the unchallenged control group. Percent necrotic enteritis mortality was significantly reduced in birds fed the algal biomass augmented feed product.

**TABLE 2: Study Results**

| | Feed Intake | | Feed Conversion | | Weight Gain | |
|---|---|---|---|---|---|---|
| Group | D0-21 | D14-21 | DO-21 | D14-21 | D0-21 | D 14-21 |
| 1. NM , No CP | 7.053a | 3.971a | 1.516b | 1.440b | 0.532a | 0.319a |
| 2. NM, CP | 7.297a | 3.654a | 1.725a | 1.745a | 0.477b | 0.240c |
| 3. Product Z 0.039%, CP | 7.213a | 3.840a | 1.561b | 1.586b | 0.527a | 0.279b |
| 4. Product Z 0.077%, CP | 6.820a | 3.651a | 1.557b | 1.594ab | 0.510ab | 0.283bc |
| 5. Product Z 0.551%, CP | 6.985a | 3.815a | 1.510b | 1.537b | 0.526a | 0.268b |
| | | | | | | |

| | Feed Intake | | Feed Conversion | | Weight Gain | |
|---|---|---|---|---|---|---|
| Group | D0-28 | D14-28 | D0-28 | D14-28 | D0-28 | D 14-28 |
| 1. NM , No CP | 9.570a | 6.488a | 1.540b | 1.501c | 0.777a | 0.564a |
| 2. NM, CP | 9.113a | 5.470c | 1.753a | 1.787a | 0.731a | 0.494a |
| 3. Product Z 0.039%, CP | 9.320a | 5.948abc | 1.609b | 1.656b | 0.794a | 0.546a |
| 4. Product Z 0.077%, CP | 9.005a | 5.837bc | 1.588b | 1.637b | 0.762a | 0.520a |
| 5. Product Z 0.551%, CP | 9.553a | 6.384ab | 1.589b | 1.646b | 0.765a | 0.522a |
| | | | | | | |

| | NE (0-3) | % NE | | | | |
|---|---|---|---|---|---|---|
| Group | Score | Mortality | | | | |
| 1. NM , No CP | 0.0b | 0d | | | | |
| 2. NM, CP | 1.1a | 25a | | | | |
| 3. Product Z 0.039%, CP | 0.8a | 13b | | | | |
| 4. Product Z 0.077%, CP | 0.9a | 9bc | | | | |
| 5. Product Z 0.551%, CP | 0.9a | 5cd | | | | |

### Example 5: Comparative Anticoccidial Efficacy of the Algal Biomass Ingredient Versus Salinomycin in Broiler Chickens

In this example, "Product Z" refers to the poultry feed that includes the algal biomass ingredient at the wt.% level shown.

### Target Animals

### Broiler chickens.

### Test Groups

1. No Additive, no Cocci.
2. No Additive, Cocci.
3. Salinomycin 60 g/t, Cocci.
4. Product Z 0.551%, Cocci.

### Experimental Design

Cages were blocked by location in the battery with block size equal to the groups (8 cages per block). The study began when the birds were placed (day of hatch, i.e., DOT 0) at which time they were allocated to the experimental cages. Only healthy birds were selected (10 birds/cage).

### Materials and Methods:

### A. Housing

Upon arrival, chicks were raised in Petersime battery cages. At placement the birds were fed the various feeds. The Petersime batteries were in an insulated building having a concrete floor and a metal overhead structure. The floor space per animal was 0.51 sq. ft. per bird. The feeder space per bird was 10 birds per 24 x 3.5-inch feeder. Uniform temperature and illumination was provided.

### B. Feeds

An unmedicated commercial chicken feed composition compounded with feedstuffs commonly used in the U.S. poultry industry, such as cereals and proteins, was obtained. The diet was an all-vegetable feed with no antibiotics, organic acids, NSP enzymes, or direct fed microbial. This feed in mash form was fed *ad libitum* from the date of chick arrival until Day 28 of the study. Experimental feeds augmented with the algal biomass were prepared from this commercial poultry feed. The feeds were mixed to assure a uniform distribution of algal biomass into the mash feed.

### C. Animals

Day of hatch male broiler chicks of strain Cobb X Cobb were obtained from Cobb-Vantress, Cleveland, GA. Sets of ten chicks were randomly selected, group weighed and placed into cages. No birds were replaced during the study.

### D. DOT 0, 14, 20, and 28 Weights

All birds were weighed by cage on DOT 0, 14, 20 and 28. Feed was weighed in on DOT 0 and remaining feed was weighed on DOT 14, 20, and 28.

### E. Oocysts Inoculation

Coccidial oocysts inoculation procedures were described. On Day 14 of the study all birds except T1 orally received a mixed *E. acervulina, E. maxima,* and *E. tenella* coccidial inoculum diluted to a 1 ml volume (p.o.). Inoculum level attempted to produce a moderate challenge.

### F. Coccidia Intestinal/Cecal Lesion Scoring

On DOT 20, five birds from each cage were selected, sacrificed, weighed, and examined for the degree of presence of coccidia lesions. If less than five birds were present at time of scoring, all remaining birds within the cage were scored. The Johnson and Reid, 1970 method of coccidiosis lesion scoring was used to score the infected region(s) of the intestine. The scoring was based on a 0 to 4 score, with 0 being normal and 4 being the most severe.

### G. Oocysts per Gram Fecal Material

Day 19 (Day 5 post challenge) drop pans from all cages were cleaned. Day 20 (Day 6 pc) mixed feces were collected from each cage. Each sample was examined for the number of oocysts per gram fecal material by fecal floatation.

### Management

1. The facility was checked thoroughly to assure that all cages had water and that feed was available in every cage. The building temperature's range was maintained at an appropriate temperature for the age of the birds.
2. Uniform and continuous illumination was provided by fluorescent lamps hung vertically along the wall.
3. Feed and water were given *ad libitum,* noting that the inclusion rate of algal biomass was fixed.
4. Cages were checked twice daily. Observations to be included were the availability of feed and water, temperature control, and any unusual conditions. The birds were watched closely for any abnormal reactions.
5. When mortality birds were removed from cages, the cage number, date, weight of the bird, sex, and probable cause of death were recorded.

### Data Analysis

Means for cage weight gain, feed consumption, feed conversion rate, lesion scores, opgs, and mortality were calculated.

### Results and Conclusions

The results appear in TABLE 3 below. Overall FCR was reduced in the algal biomass product fed group compared to the challenged control group. Birds fed the algal biomass product had similar FCR to the salinomycin (medicated) treatment group, indicating the algal biomass can be used as a natural ingredient replacement for the antibiotic.

**TABLE 3: Study Results**

| | Feed | | Wt. | |
|---|---|---|---|---|
| Day 14 | Intake | FCR | Gain | |
| 1. No Additive, Non-Infected | 2.310a | 1.155a | 0.217a | |
| 2. No Additive, Infected | 2.461a | 1.142a | 0.230a | |
| 3. Salinomycin, 60 g/t | 2.384a | 1.156a | 0.218a | |
| 4. Product Z, 0.551 % | 2.709a | 1.150a | 0.246a | |
| | | | | |

| | Feed | | Wt. | |
|---|---|---|---|---|
| Day 0-20 | Intake | FCR | Gain | |
| 1. No Additive, Non-Infected | 5.487a | 1.497c | 0.401a | |
| 2. No Additive, Infected | 4.595b | 1.768a | 0.287c | |
| 3. Salinomycin, 60 g/t | 5.433a | 1.597bc | 0.360b | |
| 4. Product Z, 0.551 % | 4.845ab | 1.647ab | 0.307c | |
| | | | | |

| | Feed | | Wt. | |
|---|---|---|---|---|
| Day 14-20 | Intake | FCR | Gain | |
| 1. No Additive, Non-Infected | 3.177a | 1.502c | 0.227a | |
| 2. No Additive, Infected | 2.134a | 2.235a | 0.100c | |
| 3. Salinomycin, 60 g/t | 3.049a | 1.744b | 0.185b | |
| 4. Product Z, 0.551 % | 2.137a | 2.112a | 0.104c | |
| | | | | |

| | Feed | | Wt. | |
|---|---|---|---|---|
| Day 0-28 | Intake | FCR | Gain | |
| 1. No Additive, Non-Infected | 5.482a | 1.554c | 0.481a | |
| 2. No Additive, Infected | 5.690a | 2.086a | 0.289c | |
| 3. Salinomycin, 60 g/t | 5.918a | 1.625bc | 0.376b | |
| 4. Product Z, 0.551 % | 5.520a | 1.766b | 0.343bc | |
| | | | | |

| | Feed | | Wt. | |
|---|---|---|---|---|
| Day 14-28 | Intake | FCR | Gain | |
| 1. No Additive, Non-Infected | 3.172ab | 1.612c | 0.306a | |
| 2. No Additive, Infected | 3.229ab | 3.132a | 0.102c | |
| 3. Salinomycin, 60 g/t | 3.534a | 1.766c | 0.202b | |
| 4. Product Z, 0.551 % | 2.812b | 2.380b | 0.140c | |
| | | | | |

| | *Eimeria* | *Eimeria* | *Eimeria* | |
|---|---|---|---|---|
| Lesion Scores | *acervulina* | *maxima* | *tenella* | Avg. |
| 1. No Additive, Non-Infected | O.Od | O.Od | O.Od | O.Od |
| 2. No Additive, Infected | 3.0a | 2.5a | 2.8a | 2.8a |
| 3. Salinomycin, 60 g/t | 2.1c | 0.9c | 0.7c | 1.3c |
| 4. Product Z, 0.551 % | 2.6b | 2.0b | 2.3b | 2.3b |
| | | | | |

| | *Eimeria* | *Eimeria* | *Eimeria* | Total |
|---|---|---|---|---|
| OPG | *acervulina* | *tenella* | *maxima* | Avg. |
| 1. No Additive, Non-Infected | 0c | 0c | 0b | 0b |
| 2. No Additive, Infected | 57112a | 23153a | 2701a | 82966a |
| 3. Salinomycin, 60 g/t | 4511c | 4727c | 484b | 9722b |
| 4. Product Z, 0.551 % | 46840a | 20835a | 2159a | 69835a |

### Example 6. Cocci Test

### Target Animals

Broiler chickens.

### Test Groups

1. No Additive, no Cocci
2. No Additive, Cocci
3. Salinomycin 60g/t, Cocci
4. Algal biomass 0.5 lbs./ton, Cocci
5. Algal biomass 2.0 lbs./ton, Cocci
6. Algal biomass 3.5 lbs./ton, Cocci
7. Algal biomass 5.0 lbs./ton, Cocci

### Experimental Design

Each group was replicated 10 times. Cages were blocked by location in the battery with block size equal to groups (7 cages per block). The study began when the birds were placed (day of hatch, i.e., DOT 0) at which time they were allocated to the experimental cages. Only healthy birds were selected (10 birds/cage).

### Materials and Methods:

### A. Housing

Upon arrival, chicks were raised in Petersime battery cages. At placement the birds were fed the various feeds. The Petersime batteries were in an insulated building having a concrete floor and a metal overhead structure. The floor space per animal was 0.51 sq. ft per bird. The feeder space per bird was 10 birds per 24 x 3.5-inch feeder. Uniform temperature and illumination was provided.

### B. Feeds

An unmedicated commercial chicken feed composition compounded with feedstuffs commonly used in the U.S. poultry industry, such as cereals and proteins, was obtained. The diet was an all-vegetable feed with no antibiotics, organic acids, NSP enzymes, or direct fed microbial. This feed in mash form was fed *ad libitum* from the date of chick arrival until Day 28 of the study. Experimental feeds augmented with the algal biomass were prepared from this commercial poultry feed. The feeds were mixed to assure a uniform distribution of algal biomass into the mash feed.

### C. Animals

Day of hatch male broiler chicks of strain Cobb 500 were obtained from Cobb-Vantress, Cleveland, GA. Sets of ten chicks were randomly selected, group weighed and placed into cages. Number and disposition of all birds not used for allocation were documented. No birds were replaced during the study.

### D. DOT 0, 14, 20, and 28 Weights

All birds were weighed by cage on DOT 0, 14, 20 and 28. Feed was weighed in on DOT 0 and remaining feed was weighed on DOT 14, 20, and 28.

### E. Oocysts Inoculation

On Day 14 of the study all birds except T1 orally received a mixed *E. acervulina, E. maxima,* and *E. tenella* coccidial inoculum diluted to a 1 ml volume (p.o.). Inoculum level attempted to produce a moderate challenge.

### F. Coccidia Intestinal/Cecal Lesion Scoring

On DOT 20, five birds from each cage were selected, sacrificed, weighed, and examined for the degree of presence of coccidia lesions. If less than five birds were present at time of scoring, all remaining birds within the cage were scored. The Johnson and Reid, 1970 method of coccidiosis lesion scoring was used to score the infected region(s) of the intestine. The scoring was based on a 0 to 4 score, with 0 being normal and 4 being the most severe.

### G. Oocysts per Gram Fecal Material

Day 19 (Day 5 post challenge) drop pans from all cages were cleaned. Day 20 (Day 6 pc) mixed feces were collected from each cage. Each sample was examined for the number of oocysts per gram fecal material by fecal floatation.

### Management

1. The facility was checked thoroughly to assure that all cages had water and that feed was available in every cage. The building temperature's range was maintained at an appropriate temperature for the age of the birds.
2. Uniform and continuous illumination was provided by fluorescent lamps hung vertically along the wall.
3. Feed and water were given *ad libitum,* noting that the inclusion rate of algal biomass was fixed.
4. Cages were checked twice daily. Observations to be included were the availability of feed and water, temperature control, and any unusual conditions. The birds were watched closely for any abnormal reactions.
5. When mortality birds were removed from cages, the cage number, date, weight of the bird, sex, and probable cause of death were recorded.

### Data Analysis

Means for cage weight gain, feed consumption, feed conversion rate, lesion scores, opgs, and mortality were calculated.

### Results and Conclusions

The results appear in TABLE 4 below. Overall body weight gain was increased when the algal biomass was fed at a level of 2.0 and 3.5 pounds algal biomass/ton finished feed compared to the no feed additive infected group. Birds fed the algal biomass at levels of 2.0 and 3.5 pounds algal biomass/ton finished feed had similar FCR to the salinomycin control group. Coccidiosis lesion score was reduced in all algal biomass fed groups compared to the no-feed additive infected group. When the algal biomass was fed above the level of 0.5 pounds algal biomass/ton finished feed, *Eimeria tenella* OPG was decreased compared to no feed additive infected group.

**TABLE 4: Study Results**

| | Feed | | Wt. | |
|---|---|---|---|---|
| Day 14 | Intake | FCR | Gain | |
| 1. No Additive, Non-Infected | 3.511a | 1.426abc | 0.248ab | |
| 2. No Additive, Infected | 3.519a | 1.454ab | 0.245ab | |
| 3. Salinomycin, 1.0 lb/t | 3.436ab | 1.491a | 0.232b | |
| 4. Algal Biomass, 0.5 lb/ton | 3.249bc | 1.361bcd | 0.239ab | |
| 5. Algal Biomass, 2.0 lb/ton | 3.429ab | 1.401abcd | 0.245ab | |
| 6. Algal Biomass, 3.5 lb/ton | 3.373ab | 1.323cd | 0.256a | |
| 7. Algal Biomass, 5.0 lb/ton | 3.118c | 1.291d | 0.242ab | |
| | | | | |

| | Feed | | Wt. | |
|---|---|---|---|---|
| Day 0-20 | Intake | FCR | Gain | |
| 1. No Additive, Non-Infected | 7.602a | 1.693c | 0.452a | |
| 2. No Additive, Infected | 7.098b | 1.952a | 0.368b | |
| 3. Salinomycin, 1.0 lb/t | 7.064b | 1.876ab | 0.382b | |
| 4. Algal Biomass, 0.5 lb/ton | 6.653d | 1.868ab | 0.363b | |
| 5. Algal Biomass, 2.0 lb/ton | 7.125b | 1.875ab | 0.381b | |
| 6. Algal Biomass, 3.5 lb/ton | 7.020bc | 1.783bc | 0.394b | |
| 7. Algal Biomass, 5.0 lb/ton | 6.683cd | 1.832abc | 0.369b | |
| | | | | |

| | Feed | | Wt. | |
|---|---|---|---|---|
| Day 14-20 | Intake | FCR | Gain | |
| 1. No Additive, Non-Infected | 4.092a | 1.610c | 0.256a | |
| 2. No Additive, Infected | 3.579bc | 2.060a | 0.175c | |
| 3. Salinomycin, 1.0 lb/t | 3.628b | 1.839b | 0.201b | |
| 4. Algal Biomass, 0.5 lb/ton | 3.404c | 2.028a | 0.176c | |
| 5. Algal Biomass, 2.0 lb/ton | 3.646b | 1.940ab | 0.188bc | |
| 6. Algal Biomass, 3.5 lb/ton | 3.647b | 1.917ab | 0.190bc | |
| 7. Algal Biomass, 5.0 lb/ton | 3.565bc | 2.040a | 0.179c | |
| | | | | |

| | Feed | | Wt. | |
|---|---|---|---|---|
| Day 0-28 | Intake | FCR | Gain | |
| 1. No Additive, Non-Infected | 10.452a | 1.606b | 0.881a | |
| 2. No Additive, Infected | 9.855bc | 1.890a | 0.666c | |
| 3. Salinomycin, 1.0 lb/t | 9.852bc | 1.832a | 0.718bc | |
| 4. Algal Biomass, 0.5 lb/ton | 9.219d | 1.836a | 0.662c | |
| 5. Algal Biomass, 2.0 lb/ton | 9.930b | 1.819a | 0.739b | |
| 6. Algal Biomass, 3.5 lb/ton | 9.802bc | 1.767a | 0.755b | |
| 7. Algal Biomass, 5.0 lb/ton | 9.516cd | 1.793a | 0.721bc | |
| | | | | |

| | Feed | | Wt. | |
|---|---|---|---|---|
| Day 14-28 | Intake | FCR | Gain | |
| 1. No Additive, Non-Infected | 6.941a | 1.522b | 0.685a | |
| 2. No Additive, Infected | 6.336b | 1.913a | 0.473c | |
| 3. Salinomycin, 1.0 lb/t | 6.416b | 1.789a | 0.537b | |
| 4. Algal Biomass, 0.5 lb/ton | 5.970c | 1.901a | 0.474c | |
| 5. Algal Biomass, 2.0 lb/ton | 6.450b | 1.823a | 0.547b | |
| 6. Algal Biomass, 3.5 lb/ton | 6.429b | 1.834a | 0.551b | |
| 7. Algal Biomass, 5.0 lb/ton | 6.398b | 1.879a | 0.531b | |
| | | | | |

| | *Eimeria* | *Eimeria* | *Eimeria* | |
|---|---|---|---|---|
| Lesion Scores | *acervulina* | *maxima* | *tenella* | Avg. |
| 1. No Additive, Non-Infected | O.Od | 0.0c | O.Od | O.Od |
| 2. No Additive, Infected | 2.9a | 2.2a | 1.7a | 2.3a |
| 3. Salinomycin, 1.0 lb/t | 1.7c | 1.7b | 1.1b | 1.5c |
| 4. Algal Biomass, 0.5 lb/ton | 2.5b | 1.7b | 1.1b | 1.8b |
| 5. Algal Biomass, 2.0 lb/ton | 2.5b | 1.6b | 1.1b | 1.7b |
| 6. Algal Biomass, 3.5 lb/ton | 2.4b | 1.7b | 0.9bc | 1.7bc |
| 7. Algal Biomass, 5.0 lb/ton | 2.5b | 1.6b | 0.8c | 1.6bc |
| | | | | |

| | *Eimeria* | *Eimeria* | *Eimeria* | Total |
|---|---|---|---|---|
| OPG | *acervulina* | *maxima* | *tenella* | Avg. |
| 1. No Additive, Non-Infected | 0c | 0b | 0c | 0c |
| 2. No Additive, Infected | 297202a | 2835a | 3469a | 303,505a |
| 3. Salinomycin, 1.0 lb/t | 30802c | 2608a | 1768b | 35,178c |
| 4. Algal Biomass, 0.5 lb/ton | 186387ab | 2348a | 2555ab | 191,289ab |
| 5. Algal Biomass, 2.0 lb/ton | 147013b | 2828a | 1521bc | 151,362b |
| 6. Algal Biomass, 3.5 lb/ton | 91693bc | 2168a | 1454bc | 953,14bc |
| 7. Algal Biomass, 5.0 lb/ton | 30802c | 2601a | 1708b | 35,111c |

## Claims

1. A poultry feed composition including an algal biomass comprising a species of *Klebsormidium* algae for use in a method of mitigating symptoms relating to coccidiosis or necrotic enteritis in poultry.

2. The poultry feed composition for use according to claim 1, wherein the *Klebsormidium* algae species is selected from the group consisting of *K. acidophilum, K. bilatum, K. crenulatum, K. dissectum, K. drouetii, K. elegans, K. flaccidum, K. fluitans, K. fragile, K. klebsii, K. lamellosum, K. montanum, K. mucosum, K. nitens, K. pseudostichococcus, K. scopulinum, K. sterile, K. subtile, K. subtilissimum, K.* tribonematoideum, and mixtures thereof.

3. The poultry feed composition for use according to claim 1 or claim 2, wherein the *Klebsormidium* algae species is selected from the group consisting of *Klebsormidium nitens, Klebsormidium flaccidum,* and mixtures thereof.

4. The poultry feed composition for use according to any one of the preceding claims, wherein the algal biomass is obtained by skimming algae from a raceway pond of continuously cultivated *Klebsormidium* algae, draining off liquid from the algae and spray drying to obtain the algal biomass.

5. The poultry feed composition for use according to any one of the preceding claims, wherein the algal biomass is present in the poultry feed composition at a level of from about 0.01% to about 1.0% by weight, based on the total weight of the composition.

6. The poultry feed composition for use according to any one of the preceding claims, wherein the poultry feed composition further comprises at least one of a cereal, protein, fat, and oil.

7. The poultry feed composition for use according to any one of the preceding claims, wherein the poultry feed composition is in the physical form of a mash feed or a pelleted feed.

8. The poultry feed composition for use according to any one of the preceding claims, wherein the symptoms include at least one of elevated feed conversion rate, reduced growth rate, increased mortality rates, and increased intestinal lesions.

## Patentansprüche

1. Eine Geflügelfutterzusammensetzung, die eine Algenbiomasse umfasst, die eine Spezies von *Klebsormidium-*Algen zur Verwendung in einem Verfahren zum Abschwächen von Symptomen in Verbindung mit Kokzidiose oder nekrotischer Enteritis bei Geflügel beinhaltet.

2. Geflügelfutterzusammensetzung zur Verwendung nach Anspruch 1, wobei die Klebsormidium-Algenspezies aus der Gruppe ausgewählt ist, die aus *K. acidophilum, K. bilatum, K. crenulatum, K. dissectum, K. drouetii, K. elegans, K. flaccidum, K. fluitans, K. fragile, K. klebsii, K. lamellosum, K. montanum, K. mucosum, K. nitens, K. pseudostichococcus, K. scopulinum, K. sterile, K. subtile, K. subtilissimum, K. tribonematoideum* und Mischungen davon besteht.

3. Geflügelfutterzusammensetzung zur Verwendung nach Anspruch 1 oder Anspruch 2, wobei die *Klebsormidium-*Algenspezies aus der Gruppe ausgewählt ist, die aus *Klebsormidium* nitens, *Klebsormidium flaccidum* und Mischungen davon besteht.

4. Geflügelfutterzusammensetzung zur Verwendung nach einem der vorhergehenden Ansprüche, wobei die Algenbiomasse erhalten wird, indem Algen von einem Langstrombecken von kontinuierlich kultivierten *Klebsormidium*-Algen abgeschöpft werden, Flüssigkeit von den Algen abgezogen wird und die Algen sprühgetrocknet werden, um die Algenbiomasse zu erhalten.

5. Geflügelfutterzusammensetzung zur Verwendung nach einem der vorhergehenden Ansprüche, wobei die Algenbiomasse in der Geflügelfutterzusammensetzung, bezogen auf das Gesamtgewicht der Zusammensetzung, in einem Anteil von etwa 0,01 bis etwa 1,0 Gewichts-% vorhanden ist.

6. Geflügelfutterzusammensetzung zur Verwendung nach einem der vorhergehenden Ansprüche, wobei die Geflügelfutterzusammensetzung ferner mindestens eines von einem Getreide, Protein, Fett und Öl beinhaltet.

7. Geflügelfutterzusammensetzung zur Verwendung nach einem der vorhergehenden Ansprüche, wobei die Geflügelfutterzusammensetzung in der physikalischen Form von Breifutter oder pelletiertem Futter vorliegt.

8. Geflügelfutterzusammensetzung zur Verwendung nach einem der vorhergehenden Ansprüche, wobei die Symptome mindestens eines von erhöhter Futterumwandlungsrate, reduzierter Wachstumsrate, erhöhten Mortalitätsraten und erhöhten Darmläsionen umfassen.

## Revendications

1. Composition d'aliment pour volailles contenant une biomasse algale comprenant une espèce d'algue *Klebsormidium* destinée à une utilisation dans une méthode d'atténuation de symptômes liés à la coccidiose ou à l'entérite nécrotique chez des volailles.

2. Composition d'aliment pour volailles destinée à une utilisation selon la revendication 1, l'espèce d'algue *Klebsormidium* étant sélectionnée dans le groupe constitué de *K. acidophilum, K. bilatum, K. crenulatum, K. dissectum, K. drouetii, K. elegans, K. flaccidum, K. fluitans, K. fragile, K. klebsii, K. lamellosum, K. montanum, K. mucosum, K. nitens, K. pseudostichococcus, K. scopulinum, K. stérile, K. subtile, K. subtilissimum, K. tribonematoideum,* et de mélanges de celles-ci.

3. Composition d'aliment pour volailles destinée à une utilisation selon la revendication 1 ou la revendication 2, l'espèce d'algue *Klebsormidium* étant sélectionnée dans le groupe constitué de *Klebsormidium nitens, Klebsormidium flaccidum,* et de mélanges de celles-ci .

4. Composition d'aliment pour volailles destinée à une utilisation selon l'une quelconque des revendications précédentes, dans laquelle la biomasse algale est obtenue en récoltant des algues à la surface d'un bassin à ciel ouvert de culture continue d'algues *Klebsormidium,* égouttage des algues et séchage par atomisation pour obtenir la biomasse algale.

5. Composition d'aliment pour volailles destinée à une utilisation selon l'une quelconque des revendications précédentes, la biomasse algale étant présente dans la composition d'aliment pour volailles à un niveau d'environ 0,01 % à environ 1,0 % en poids, sur la base du poids total de la composition.

6. Composition d'aliment pour volailles destinée à une utilisation selon l'une quelconque des revendications précédentes, la composition d'aliment pour volailles comprenant en outre au moins l'une d'une céréale, d'une protéine, d'une matière grasse et d'une huile.

7. Composition d'aliment pour volailles destinée à une utilisation selon l'une quelconque des revendications précédentes, la composition d'aliment pour volailles étant sous la forme physique d'un aliment en purée ou d'un aliment en granulés.

8. Composition d'aliment pour volailles destinée à une utilisation selon l'une quelconque des revendications précédentes, les symptômes comprenant au moins l'un d'un indice de consommation élevé, d'un taux de croissance réduit, de taux de mortalité accrus, et d'une augmentation des lésions intestinales.
